**Europäisches Patentamt**

**European Patent Office**

**Office européen des brevets**

(19)

(11) Veröffentlichungsnummer : **0 016 480 B1**

(12) **EUROPÄISCHE PATENTSCHRIFT**

(45) Veröffentlichungstag der Patentschrift :
16.02.83

(51) Int. Cl.³ : **A 61 F   1/00**

(21) Anmeldenummer : **80200090.1**

(22) Anmeldetag : **01.02.80**

(54) Strumpfartiger Überzug für den Verankerungsschaft einer Endoprothese.

(30) Priorität : **15.03.79 CH 2439/79**

(43) Veröffentlichungstag der Anmeldung :
**01.10.80 Patentblatt 80/20**

(45) Bekanntmachung des Hinweises auf die Patenterteilung : **16.02.83 Patentblatt 83/07**

(84) Benannte Vertragsstaaten :
**AT DE FR GB SE**

(56) Entgegenhaltungen :
**DE A 2 206 144**
**DE A 2 502 884**
**DE A 2 617 749**
**DE A 2 628 284**
**DE A 2 802 295**
**DE B 2 205 808**
**DE B 2 334 643**
**US A 4 064 567**

(73) Patentinhaber : **GEBRÜDER SULZER AKTIENGE-SELLSCHAFT**
**Zürcherstrasse 9**
**CH-8401 Winterthur (CH)**

(72) Erfinder : **Weber, Bernhard Georg Prof. Dr.-med.**
**Kantonsspital**
**CH-9000 St. Gallen (CH)**

(74) Vertreter : **Dipl.-Ing. H. Marsch Dipl.-Ing. K. Sparing Dipl.-Phys.Dr. W.H. Röhl Patentanwälte**
**Rethelstrasse 123**
**D-4000 Düsseldorf (DE)**

## Strumpfartiger Ueberzug für den Verankerungsschaft einer Endoprothese

Die Erfindung betrifft einen strumpfartigen Ueberzug für den Verankerungsschaft einer Endoprothese, der mittels Knochenzement in einen menschlichen Knochen verankert wird, wobei ein den Schaft lose umhüllender, schmiegsamer, in Achsrichtung des Schaftes streckbarer und in Umfangsrichtung erweiterbarer Beutel vorgesehen ist, der an seinem offenen Ende eine verengte Durchstecköffnung für den Prothesenschaft aufweist und flüssigkeitsdicht ausgebildet ist.

Sowohl die bisherige Art der Verankerung eines Prothesenschaftes mit Knochenzement als auch das zementfreie Einsetzen der Prothesen besitzen neben ihren spezifischen Vorteilen eine Reihe von Nachteilen. So ist eine zementfreie Verankerung, die eine hohe Genauigkeit an die Anpassung zwischen Implantatschaft und dem operativ ausgeräumten Knochen erfordert, nur bei etwa 20 bis 30 % der Implantationen möglich, da für sie der Knochen noch regenerierfähig, aktiv und vital sein muss, um ein festes Einwachsen innerhalb zumutbarer Zeiträume der Ruhigstellung zu erreichen.

Die bekannten Nachteile der Verwendung von Knochenzementen sind in erster Linie die bei der Polymerisation dieser Zemente auftretende Wärmeentwicklung, die zu lokalen Zerstörungen des umgebenden Knochengewebes führen kann und die Abgabe von im Körper toxisch wirkenden Stoffen. Weiter hat sich gezeigt, dass es in den äusseren, zum Knochengewebe hin gelegenen Berichen von Zementköchern nicht mehr zu einer Bindung zwischen den einzelnen, kugelartigen Polymerpartikeln kommt, so dass die äussere Oberfläche der Zementbetten zerrüttet ist. Der Grund für diese mangelhafte Bindung der Teilchen liegt darin, dass das flüssige, durch Polymerisation die Bindung bewirkende Monomer aus den Zwischenräumen zwischen den Polymerteilchen durch Körperflüssigkeit verdrängt wird.

Es ist daher bekannt (DE-B-23 34 643), das Knochengewebe und den Zement für die Verankerung des Schaftes durch eine resorbierbare Folie voneinander flüssigkeitsdicht zu trennen. Aus medizinischen Gründen ist jedoch die Verwendung resorbierbarer Folien bei der Verankerung von Prothesenschäften im Knochen nicht erwünscht, denn bei rascher Resorption besteht die Gefahr, dass sich zwischen Knochen und dem Zementbett Hohlräume bilden, die die Stabilität der Verankerung beeinträchtigen ; Aufgabe der Erfindung ist es, eine solche quasi-zementfreie Verankerung zu schaffen, ohne die Stabilität zu gefährden, und darüberhinaus eine innige Verbindung des Beutels mit dem umgebenden Knochengewebe zu gewährleisten. Die Lösung dieser Aufgabe erfolgt gemäss der Erfindung dadurch, dass der Beutel aus einem körperbeständigen Material besteht und auf seiner Aussenseite eine, das Einwachsen von Gewebe fördernde Oberflächenstruktur aufweist.

Aus der DE-B-2 205 808 ist es an sich bekannt, bei Knochenimplantaten den Schaft selbst mit einer Vertiefungen aufweisenden Oberflächenstruktur zu versehen, um das einwachsen von Gewebe zwecks Verankerung zu ermöglichen.

Die Körperbeständigkeit des Beutels verhindertauf einfache Weise die Entstehung von Hohlräumen in der Uebergangsschicht zwischen Zement und Knochengewebe ; um die dadurch verbesserte Stabilität der Verankerung zu gewährleisten, muss eine innige Haftung zwischen Beutelaussenseite und dem umgebenden Knochen sichergestellt werden. Dies gelingt dadurch, dass an den Knochen angrenzend eine Oberflächenstruktur des Beutels erzeugt wird, die das Einwachsen von Gewebe begünstigt.

Der Beutel, der mit Vorteil schlecht wärmeleitend ausgebildet ist, besteht aus einem körperverträglichen Kunststoff, beispielsweise Polyäthylen, Polyester oder vorzugsweise einem Silikonkautschuk ; seine äussere Oberflächenstruktur wird durch zottenartige Vertiefungen und/oder Vorsprünge gebildet oder durch eine offenporige Oberflächenschicht, deren Porosität nach aussen zum Knochen hin zunimmt. Die nach aussen zunehmende Porosität einer offenporigen Oberflächenschicht ist notwendig, um ein tiefes Einwachsen von Knochengewebe in diese Schicht zu erreichen und eine ausreichende Ernährung des eingewachsenen Gewebes zu gewährleisten.

Die Dicke der flüssigkeitsdichten Innenwand des Beutels kann beispielsweise 0,01 bis 0,3 mm betragen, während die strukturierte äussere Oberfläche zum Beispiel 0,1 bis 0,4 mm dick sein kann. Die Herstellung der Innenwand erfolgt beispielsweise durch ein- oder mehrfaches Tauchen eines entsprechend geformten Kerns in einer mit einem Lösungsmittel versetzten Kunststoffmasse ; ist die notwendige Dicke der Innenwand erreicht, so wird aus dem gleichen Material ein aus einer Düse gespritzter Faden auf beliebigen Wegen um die sich noch auf dem Kern befindliche Innenwand gewockelt, wobei der Faden zunächst an der noch nicht getrockneten Innenwand und später an bereits auf dem Kern haftenden Fäden hängenbleibt. Auf diese Weise kann z. B. eine poröse Oberflächenschicht mit offenen Poren erzeugt werden ; wird der zum Tauchen benutzte Kern selbst an seiner Oberfläche mit einer Struktur versehen, so bildet sich diese selbstverständlich auf der Innenwand des Beutels ab.

Die Verwendung des erfindungsgemässen Ueberzugs kann beispielsweise so erfolgen, dass der Beutel zunächst in den vorbereiteten Operationskanal eingeführt und mit einem geeigneten Instrument in etwa an die Begrenzungen der Operationsöffnung angelegt wird. Anschliessend wird der Beutel mit Knochenzement gefüllt ; durch das darauffolgende Einsetzen und Einschlagen des Prothesenschaftes wird der im Beutel befindliche Zement nach allen Seiten verdrängt, wobei der schmiegsame und in Umfangsrichtung erweiterbare, sowohl in Achsrichtung der Operationsöffnung streckbare Beutel, den Unebenheiten der Begrenzungen der Operationsöffnung nachgebend, sich in allen Richtungen an diese Begrenzungen anschmiegt. Dabei ist es selbstverständlich nicht notwendig, dass der Beutelumfang und seine Länge exakt an die Operationsöffnung angepasst sind. Sollte seine Weite und seine Länge zu gross sein, so ist es ohne weiteres möglich, dass er Falten bildet ; zu geringe Weiten und Längen können durch plastische und/oder elastische Dehnungen seines Materials in gewissem Umfang ausgeglichen werden.

Der flüssigkeitsdichte Beutel verhindert weiterhin nicht nur jeden Kontakt des Knochengewebes mit den toxischen Komponenten des Zements, sondern verzögert aufgrund seiner Wärmeisolationswirkung den Wärmefluss in das Gewebe hinein, so dass die bei der Polymerisation nur kurzzeitig entstehende Wärme vermehrt über den Implantatschaft, der vorzugsweise aus Metall besteht, abfliessen kann, ehe sie Hitzeschäden in dem umgebenden Knochengewebe verursacht.

Im folgenden wird die Erfindung anhand von Ausführungsbeispielen im Zusammenhang mit der Zeichnung näher erläutert.

Figur 1 ist ein Längsschnitt durch einen Oberschenkelknochen, in dem der Schaft einer Hüftgelenksprothese mit Hilfe des erfindungsgemässen Ueberzugs verankert ist ;

Figur 2 gibt das Details A von Fig. 1 in grösserem Massstab wieder ;

Figur 3 ist eine, räumlich gesehen, teilweise im Schnitt dargestellte Skizze eines für den Schaft eines Hüftgelenkes bestimmten Ueberzugs und

Figur 4 schliesslich ist eine ähnliche Skizze wie Fig. 3 und zeigt einen Ueberzug, der für den Schaft eines anderen Gelenkimplantates, beispielsweise für ein Kniegelenk, bestimmt ist.

Der Schaft 1 einer an sich bekannten Hüftelenk-Rotationsprothese sitzt im spongiosen Gewebe 2 eines Oberschenkelknochens, dessen kortikale Aussenschale mit 3 bezeichnet ist.

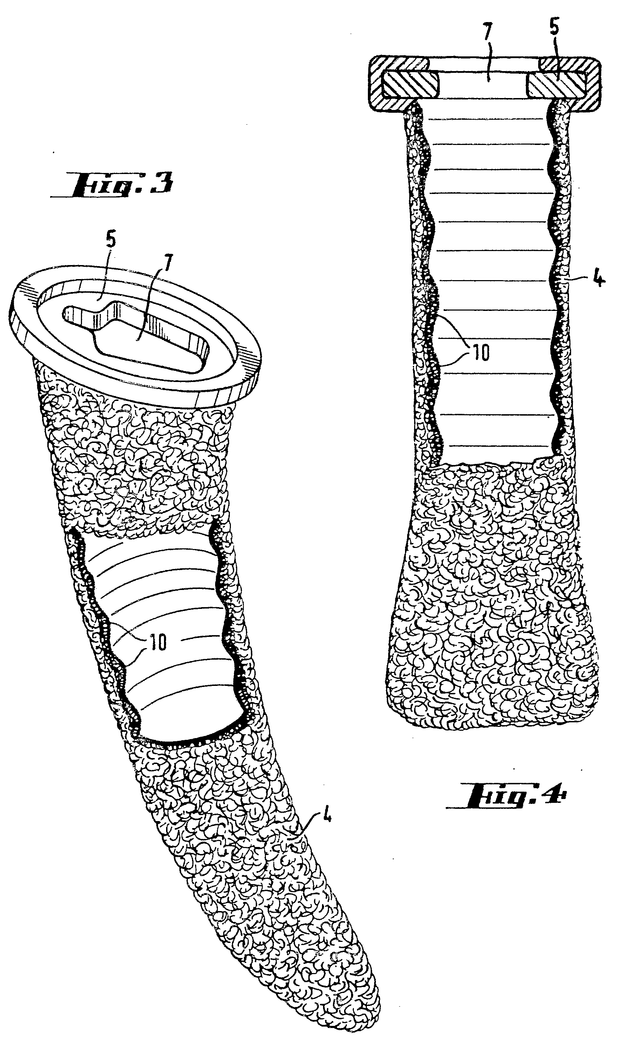

In die für die Aufnahme des Schaftes 1 operativ vorbereitete Spongiosa 2 ist dabei zunächst ein strumpfartiger Ueberzug eingesetzt, dessen Beutel 4 an seinem oberen Ende eine Abdeck- oder Trochanterplatte 5 umschlingt und an dieser durch eine Naht 6 bzw. durch Schweissen oder Kleben befestigt ist. Wie Fig. 3 zeigt, hat die Trochanterplatte 5, die sich vorwiegend auf dem tragfähigen, kortikalen Gewebe 3 abstützt, eine in Form und Grösse an das gelenkseitige Ende des Prothesenschaftes 1 angepasste Durchstecköffnung 7.

Zwischen dem Schaft 1 und dem Beutel 4 ist ein Knochenzementbett 8 vorhanden, in dem der Schaft 1 festgehalten ist.

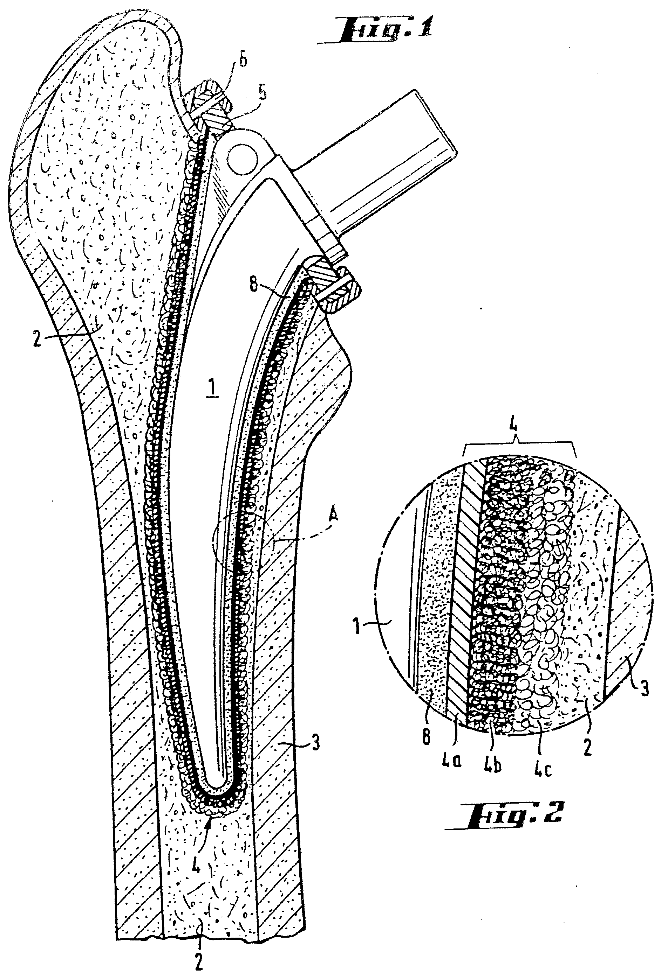

Im Querschnitt des Beutels 4 lassen sich, wie aus Fig. 2 ersichtlich, drei verschiedene Schichten unterscheiden : Eine Innenschicht 4a, die die flüssigkeitsdichte Innenwand des Beutels 4 bildet, eine bereits mit Poren versehene, jedoch noch relativ dichte Zwischenschicht 4b, und schliesslich aussen, d. h. zum Knochengewebe hin, eine äussere Oberflächenschicht 4c, die durch eine Vielzahl von Poren charakterisiert ist. In diese strukturierte äussere Oberflächenschicht 4c wächst im Laufe der Zeit Knochengewebe ein, womit eine feste Verankerung des Ueberzugs im Knochen entsteht.

Um die Haftung zwischen dem Knochenzement 8 und der Beutelinnenwand 4a zu verbessern, kann diese Innenwand ebenfalls eine Struktur 10 besitzen, die in den in Fig. 3 und 4 gezeigten Beispielen in einer, im wesentlichen quer zur Schaftachse verlaufenden, wellenartigen Ausbildung besteht.

Der in Fig. 4 gezeigte Ueberzug ist für den Femurteil einer Kniegelenkprothese bestimmt ; bei dieser ist die Weite des operativ eröffneten Knochens im Kondylenbereich kleiner als weiter im Innern des Femurs. Diesem Umstand ist bei dem Ausführungsbeispiel des Ueberzugs nach Fig. 4 Rechnung getragen, indem sich der Beutel zu seinem geschlossenen Ende hin nich, wie in Fig. 1 und 3, konisch verengt, sondern im Gegenteil konisch erweitert.

## Ansprüche

1. Strumpfartiger Ueberzug für den Verankerungsschaft (1) einer Endoprothese, der mittels Knochenzement in einen menschlichen Knochen verankert wird, wobei ein den Schaft (1) lose umhüllender, schmiegsamer, in Achsrichtung des Schaftes (1) streckbarer und in Umfangsrichtung erweiterbarer Beutel (4) vorgesehen ist, der an seinem offenen Ende verengte Durchstecköffnung für den Prothesenschaft (1) aufweist und flüssigkeitsdicht ausgebildet ist, dadurch gekennzeichnet, dass der Beutel (4) aus einem körperbeständigen Material besteht, und auf seiner Aussenseite (4b, 4c) eine, das Einwachsen von Gewebe (2) fördernde Oberflächenstruktur aufweist.

2. Ueberzug nach Anspruch 1, dadurch gekennzeichnet, dass die Oberflächenstruktur in einer offenporigen Oberflächenschicht (4b, 4c) besteht, deren Porosität sich von der flüssigkeitsdichten Innenschicht (4a) aus nach aussen erhöht.

3. Ueberzug nach Anspruch 1, dadurch gekennzeichnet, dass die Oberflächenstruktur in zottenartigen Vertiefungen und/oder Vorsprüngen besteht.

## Claims

1. A stocking-like covering for the intramedullary stem (1) of an endoprosthesis anchored by means of bone cement in a human bone, the stem (1) having around it a loose flexible bag (4) which is extensible axially of the stem (1) and which can widen peripherally, is formed at its open end with a narrow push-through aperture for the stem (1) and is liquid-tight, characterised in that the bag (4) is made of a material resistant to the human body and has on its outside (4b, 4c) a surface structure promoting fibrous encapsulation (2).

2. A covering according to claim 1, characterised in that the surface structure takes the form of an openpored surface layer (4b, 4c) whose porosity increases outwardly from the liquid-tight inner layer (4a).

3. A covering according to claim 1, characterised in that the surface structure takes the form of villuslike recesses and/or projections.

**Revendications**

1. Gaine sous forme de bas pour la tige d'ancrage (1) d'une endoprothèse, qui est ancrée au moyen de ciment pour os dans un os humain, tandis qu'on prévoit un sac (4) souple, entourant la tige (1) de façon lâche, extensible dans le sens axial de la tige (1) et pouvant s'agrandir dans la direction périphérique, qui présente à son extrémité ouverte un orifice rétréci d'insertion pour la tige de prothèse (1) et qui est conformé de manière à être étanche aux liquides, gaine caractérisée en ce que le sac (4) est constitué d'une matière corporellement inaltérable et présente sur son côté externe (4b, 4c) une structure de surface favorisant la croissance de tissu (2).

2. Gaine selon la revendication 1, caractérisée en ce que la structure de surface consiste en une couche de surface (4b, 4c) à pores ouverts dont la porosité augmente vers l'extérieur à partir de la couche interne (4a) étanche aux liquides.

3. Gaine selon la revendication 1, caractérisée en ce que la structure de surface consiste en cavités et/ou protubérances du type en touffe.

*Fig:1*

*Fig:2*

*Fig. 3*

*Fig. 4*